Europäisches Patentamt

European Patent Office

Office européen des brevets

(19)

(11) Publication number: **0 034 479**

**A1**

## (12) EUROPEAN PATENT APPLICATION

(21) Application number: **81300594.9**

(22) Date of filing: **13.02.81**

(51) Int. Cl.³: **A 61 B 7/04**

(30) Priority: **14.02.80 US 121361**
**27.01.81 US 227611**

(43) Date of publication of application:
**26.08.81 Bulletin 81/34**

(84) Designated Contracting States:
**DE FR GB IT NL**

(71) Applicant: **ORGANON TEKNIKA CORPORATION**

**Oklahoma Oklahoma(US)**

(72) Inventor: **Smith, Damon**
**1800 Williamsburg Road Apartment 1-C**
**Durham, N.C. 27707(US)**

(74) Representative: **Boon, Graham Anthony**
**Elkington and Fife High Holborn House 52/54 High Holborn**
**London, WC1V 6SH(GB)**

(54) **Cardiac monitoring system.**

(57) A cardiac monitoring system includes a high fidelity transducer (19) wherein the body of the transducer (19) incorporates a rim (23) which contacts the skin of the chest wall of the patient. The transducer itself is restrained or fixed relative to the back of the patient. This allows signals, for example signals relating to valvular events, to be read from the heart which heretofore have not been obtainable in noninvasive cardiological monitoring.

Fig. 1

EP 0 034 479 A1

- 1 -

CARDIAC MONITORING SYSTEM

This invention relates to a cardiac monitoring system.

Since ancient times it has been recognized by physicians that the heart of a patient can be monitored by feeling and/or listening to the chest of the patient. In recent times, cardiologists have been interested in the vibrations which emanate from the heart and reach the surface of the skin to aid in assessing the function of the heart as it goes through the cardiac cycle of contraction and relaxation. These vibrations are, of course, audible with the use of a stethescope and can be sensed electronically to produce a graphical record of vibrations.

Rather sophisticated electronic equipment has been developed for use in cardiac examination units and similar locations using high fidelity transducers which typically are of the electrodynamic or crystal type and are capable of responding to vibrations with frequencies up to several thousand cycles per second. The body of the transducer is allowed to ride with the chest wall so that the sensor responds to the relative movement of the skin of the wall with respect to the transducer body. This relative movement depends on the mass of the transducer, the physical characteristics of the skin and underlying subcutaneus tissue of the chest wall, and the frequency of the vibrations of the skin. Because it is not possible to interpret the signal which results from the transducers as being a distinct parameter of motion of the skin such as positional change, velocity or acceleration, no attempt at such distinction has been made. In most of these prior devices the signal from the transducer is filtered

- 2 -

by a high pass filter so that the frequency response of the system approximates the response of the human ear and the signal thus obtained (called a phonocardiogram) is recorded on strip chart paper along with other pertinent signals associated with the heart such as an electrocardiogram.

The waveforms obtained from the above have been thought to contain vibrations originating primarily from the closing of the heart valves. On occasion various pathological states of the cardiac organ produce additional vibrations which are helpful to the attending physician in diagnosis. Detail information as to the valve openings as well as other significant cardiac vibrations have not, however, heretofore been assertable or recordable.

After much research and study into the above-mentioned problems, it has been determined that a significant improvement of the informational quality of the vibrations of cardiac origin can be obtained through constraining the transducer from moving with the chest wall instead of riding freely therewith. This constraint allows the sensor of the transducer to respond solely to the movement of the skin of the chest wall and not to the relative movement of the skin with respect to the transducer body. The signal from the transducer can thus be identified as a distinct parameter of motion of the skin such as velocity.

Accordingly, the present invention provides a cardiac monitoring system comprising: a high fidelity vibration sensing means; means for supporting said sensing means adjacent the exterior of the chest cavity of a patient in spaced, relative fixed relationship on the back of such patient; visual display means; and means operatively connecting said sensing means to said display means whereby the cardiac organ of the patient can be monitored in detail.

A rim or shield is preferably incorporated into the transducer body and is in direct contact with the chest wall. This rim or shield isolates the skin enclosed by the same from artifactual vibrations of noncardiac origin which may from time to time propagate across the skin of the chest wall. It also compresses the soft subcutaneous tissue of the chest wall to allow subtle high frequency cardiac vibrations to travel through such soft tissue more efficiently to be sensed more accurately by the cardiac monitor. Thus by fixing the body of the transducer rigidly so that it is constrained against movement with the chest wall, combined with shielding the skin below the transducer from non-cardiac vibrations, high fidelity cardiac vibrations can be recorded which allow monitoring and study of distinct valve openings and closings as well as other significant cardiac events.

In the accompanying drawings:

Figure 1 is an elevational view of a patient with the transducer portion of the cardiac monitoring system of the present invention operatively associated therewith;

Figure 2 is a strip chart produced by a prior art monitor showing a phonocardiogram with an electrocardiogram therebeneath;

Figure 3 is a strip chart showing the detailed information obtainable with the cardiac monitoring system of the present invention and its relationship to a standard electrodcardiogram;

- 4 -

Figure 4 is a strip chart showing simultaneous recordings using both the present invention and three other cardiac monitors; and

Figure 5 is an enlarged, partially cut-away elevational view of the transducer and its associated parts.

With further reference to the drawings, a patient 10 is shown lying on a table or bed 11 supported by means such as legs 12. A bracket or similar means 13 is fixedly secured to table or bed 11 and is adapted to removably receive a transducer support arm 14. A securing means such as thumb screw 15 is operatively associated with the bracket 13 for releasably holding support arm 14.

At the end of support arm 14 opposite bracket 13 is an adjusting collar 16 which is adapted to slidingly receive transducer support column 17. A thumb screw 18 is provided in adjusting collar 16 for releasably securing arm 14 to column 17.

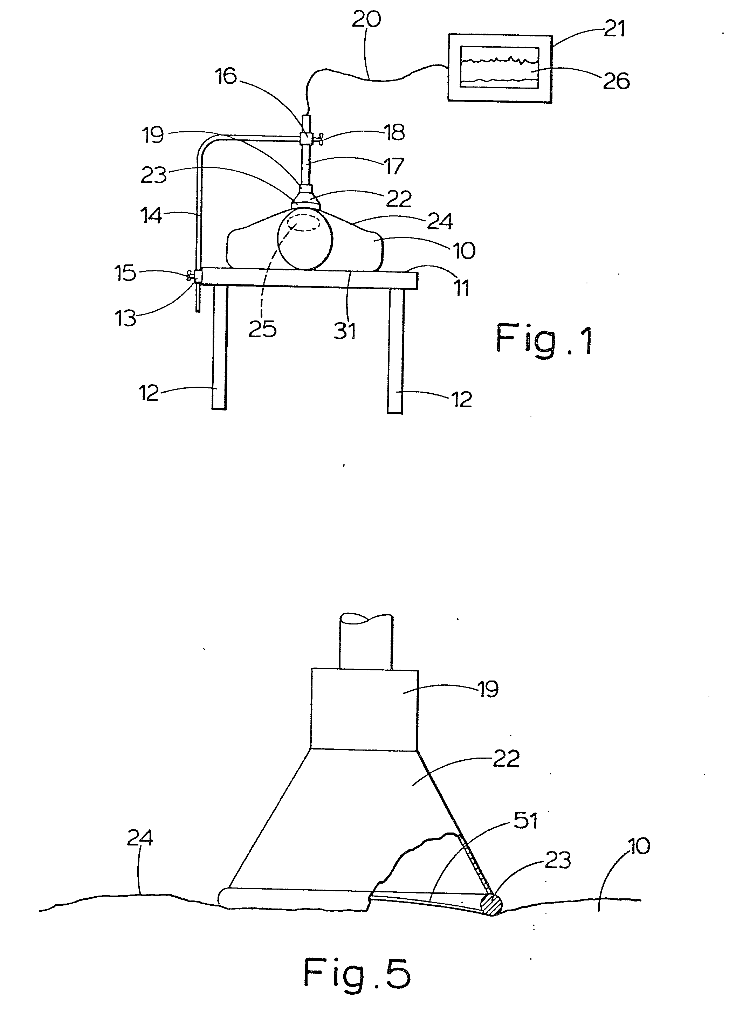

A transducer 19, preferably of either the electrodynamic or crystal type, is operatively mounted on the lower end of column 17 as disposed in Fig. 1. This transducer is operatively connected through line 20 to a strip recorder 21. This recorder can be of any one of a number of devices currently available to the medical profession.

Connected to the lower portion of transducer 19 is a preferably conical member 22 with a rim or edge 23 adapted to lie juxtaposed to the skin 24 of the chest wall above the cardiac organ 25 of patient 10.

When the cardiac monitoring system of the present invention is in operation, the transducer 19 will pick up vibratory signals from the cardiac organ 25 and transmit such signals through line 20 to strip recorder 21 where such vibrations will be displayed graphically on strip chart 26.

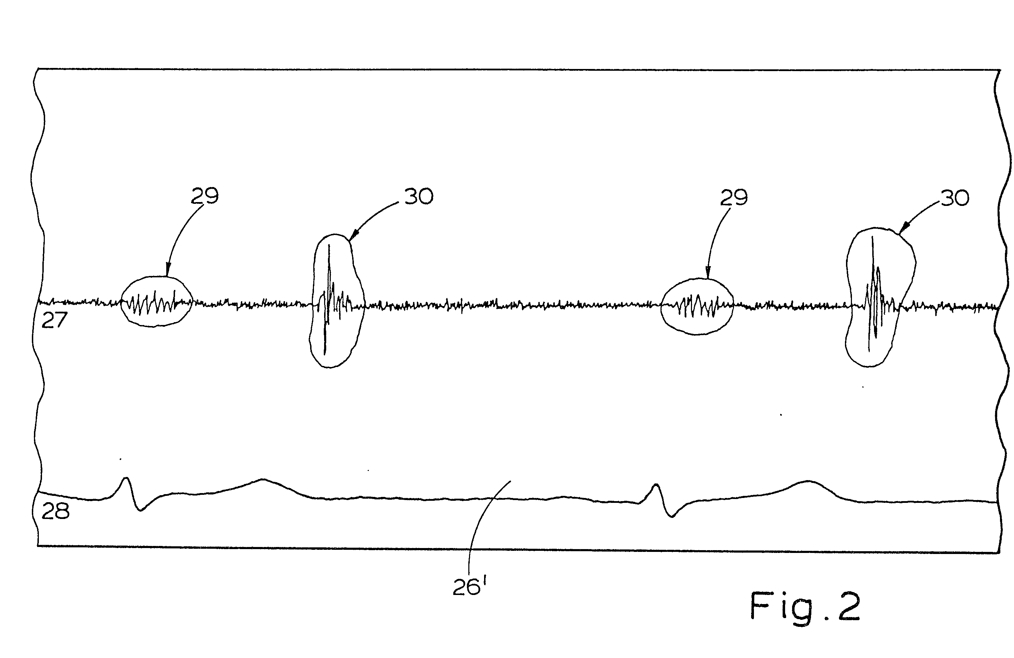

A prior art strip chart 26' is shown in Fig. 2 with a phonocardiogram being indicated at 27 and an electrocardiogram being indicated at 28. The two sounds indicated graphically at 29 and 30 of phonocardiogram 27 represent what is commonly called the first heart sound and second heart sound, respectively. Although both of these sounds are composed of vibrations of cardiac origin, it is not possible to separate the wave form into distinct events such as mitral and tricuspid valve closures which are presumed to make up the first heart sound 29 or the aortic and pulmonary valve closures which are presumed to make up the second heart sound 30. The electrocardiogram was recorded simultaneously with the phonocardiogram on strip chart 26' and is used for reference purposes.

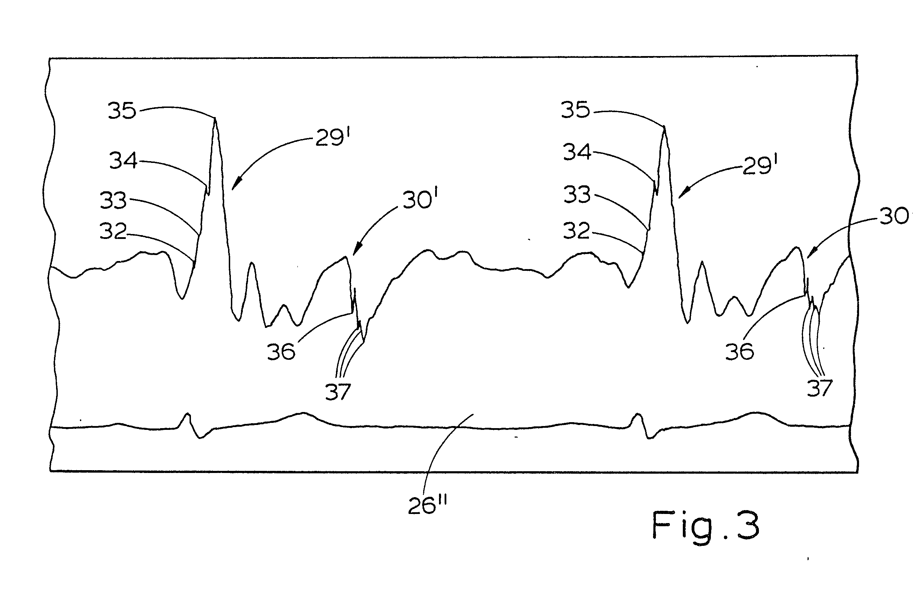

A similar electrocardiogram is also shown on strip chart 26'' in Fig. 3, so that the prior strip chart and the strip chart of the present invention can be readily compared. Referring more specifically to strip chart 26'' of Fig. 3, which is a high fidelity record of skin velocity of the chest wall taken at the same location as strip chart 26', the first heart sound indicated at 29' and the second heart sound indicated at 30' show significant improvement in the informational quality of these signals. With the transducer being held in fixed relationship to the opposite side or back 31 of patient 10 through arm 14, coupled with the use of rim 23 to eliminate random skin vibrations, it is possible to obtain the improved Fig. 3 chart. From extensive research, it appears that the vibration indicated at 32 is the mitral valve closure, 33 is the tricuspid valve closure, 34 is the pulmonary valve opening and 35 is the aortic valve opening.

In the second heart sound, it appears that 36 is the aortic valve closure and 37 is the pulmonary valve closure. The opening of the mitral and tricuspid valves are of such weak signal that these do not appear to be recordable at the outer skin level of the patient with the sensor at the present location. As can be seen from the electrocardiogram at the bottom of chart 26'', valve openings and closings are repetitive for each cycle of the cardiac organ.

Through the use of a transducer of the electrodynamic, piezoelectric crystal, condenser or strain gauge type disposed adjacent the chest of the patient and held in fixed relationship to the back of the patient, coupled with the random vibration dampening rim, significantly enhanced high fidelity recordings of the functioning cardiac organ can be obtained by noninvasive means.

Although not specifically shown, it is to be understood that the transducer 19 can be either air coupled, mechanically coupled or otherwise associated with the skin of the chest wall so that the vibration of the cardiac organ can be received and transmitted to the recorder.

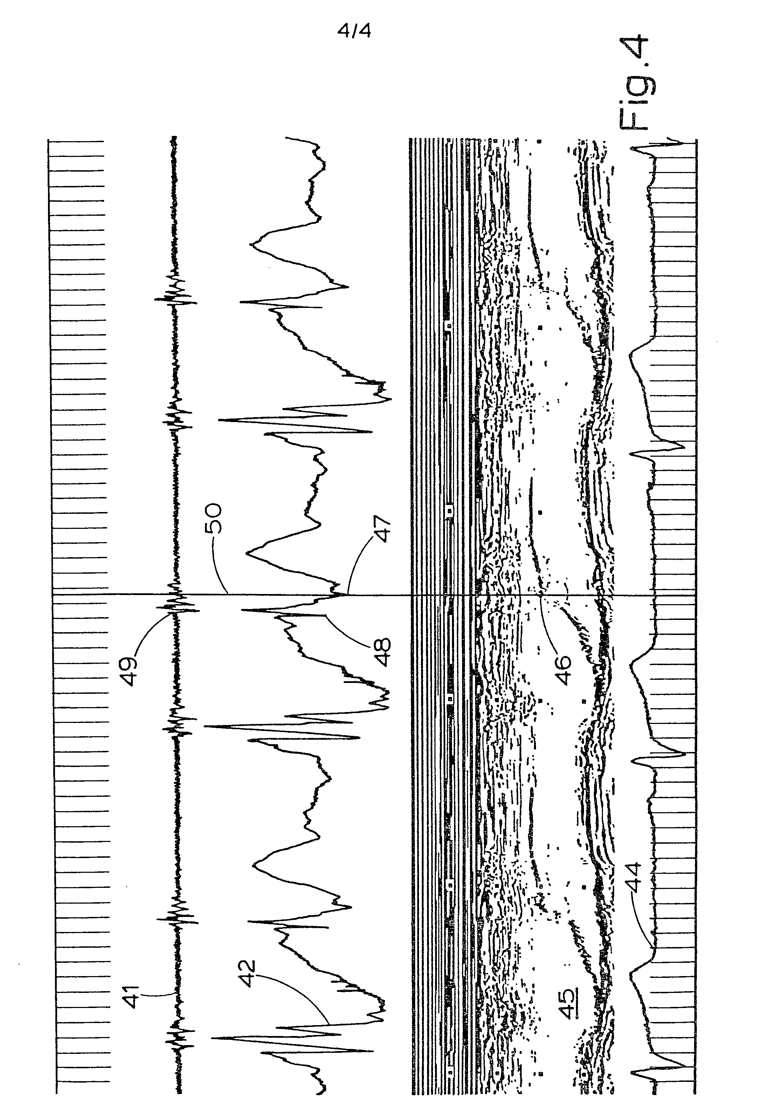

With respect to Figure 4, the velocity of the chest wall motion at the third left intercostal space at the sternal edge as monitored by the system of the present invention is shown at 42 and is simultaneously recorded with a conventional phonocardiogram at the pulmonary area indicated at 41. Also an echocardiogram is indicated at 45 and is the relative wide recording in which the movement of one of the leaflets of the pulmonary valve is shown at 46. An electrocardiogram has also been simultaneously recorded for timing reference and is indicated at 44.

It can be seen from Figure 4 that the downward moving velocity spike 48 corresponds to the beginning of vibration 49 in the phonocardiogram and is known to be the aortic closure component of the second heart sound. The second downward moving spike 47 of the velocity signal is known to be the pulmonary closure component of the second heart sound although this event fails to be identified in the phonocardiogram 41.

The vibration isolation shield or rim 23 enables the simultaneous use of such monitor with an echocardiograph even though the two transducers must be placed very close together on the chest wall. The isolation rim 23, as previously mentioned, prevents artificial vibrations of the surface of the skin due to the hand-held echocardiograph transducer from reaching the enclosed region of the chest below the rim which is sensed by the cardiac monitor of the present invention. The signal from the echocardiograph is shown at 45. The movement of one of the leaflets of the pulmonary valve is shown in the act of closing in which the instant of closure is identified as point 46 in the echocardiograph 45. This event is seen by added perpendicular reference line 50 to be synchronous with velocity spike 47 of signal 42.

In addition to the improved signal quality obtained by use of the vibration isolation shield or rim 23, an enhancement of the cardiac information reaching the surface of the skin can be accomplished by incorporating either across the rim of the transducer or otherwise within the housing, a lightweight, flexible membrane 51 composed of neoprene or similar material which slightly compresses the skin enclosed by the edge or rim of the shield 23. In other words, the light, flexible membrane 51 improves the vibration transmission characteristics of the soft subcutaneous tissue of the chest wall. This allows subtle high frequency cardiac vibrations to travel through the soft tissue more efficiently to be sensed by the cardiac monitor and in fact such a device was used in the recording of strip chart 42.

From the above, it is obvious that the present invention provides a relatively simple and inexpensive means for dramatically increasing the graphical display of the functioning cardiac organ. Through the use of these extremely accurate recordings, a physician can much more readily determine abnormality or disease of the valve leaflets and other cardiac problems that have, prior to the present invention, been undeterminable in noninvasive cardiology.

CLAIMS:

1. A cardiac monitoring system comprising: a high fidelity vibration sensing means; means for supporting said sensing means adjacent the exterior of the chest cavity of a patient in spaced, relative fixed relationship on the back of such patient; visual display means; and means operatively connecting said sensing means to said display means whereby the cardiac organ of the patient can be monitored in detail.

2. A system according to Claim 1 wherein the sensing means is an electrodynamic transducer.

3. A system according to Claim 1 wherein the sensing means is a piezoelectric crystal transducer.

4. A system according to Claim 1 wherein the sensing means is a condenser-type transducer.

5. A system according to Claim 1 wherein the sensing means is a strain gauge-type transducer.

6. A system according to any preceding claim, including a vibration dampening means associated with said sensing means and in contact with the exterior of said chest.

7. A system according to Claim 6 wherein the vibration dampening means is a rim-like structure.

8. A system according to Claim 7 wherein the rim-like structure is one end of a conical shaped member secured at its other end to said sensing means.

9. A system according to Claim 7 or 8, wherein a flexible membrane is disposed against the exterior of the chest within the confines of said rim-like structure.

10. A system according to Claim 9, wherein said membrane is stretched across the opening formed by said rim-like structure.

11. A system according to Claim 9 or 10, wherein said membrane is a lightweight flexible sheet of neoprene.

12. A system according to any preceding Claim, wherein the visual display means is a strip chart type recording device.

13. A cardiac monitoring system substantially as herein described with reference to Figures 1 and 3 to 5 of the accompanying drawings.

Fig.1

Fig.5

Fig.2

0034479

Fig.3

0034479

0034479

Fig.4

0034479

European Patent Office

**EUROPEAN SEARCH REPORT**

Application number

EP 81 30 0594

| **DOCUMENTS CONSIDERED TO BE RELEVANT** | | | CLASSIFICATION OF THE APPLICATION (Int Cl.³) |
|---|---|---|---|
| Category | Citation of document with indication, where appropriate, of relevant passages | Relevant to claim | |
| | GB - A - 1 219 618 (VSESOJUSNY NAUCHNO-ISSLE DOVATELSKY-INSTITUT) <br> * Page 1, lines 43-57; page 2, lines 3-79; figures 1-6 * <br> --- | 1 | A 61 B 7/04 |
| | US - A - 3 283 181 (F.R. JOHANSON) <br> * Column 1, lines 45-57; column 2, lines 16-46; column 3, lines 7-56; column 4, lines 57-68; figures 1-4 * <br> --- | 1,3 | |
| | FR - A - 2 405 060 (D.P. ATTEN-BURROW) <br> * Page 1, line 28 to page 2, line 6; page 2, line 25 to page 4, line 15; figures 1-5 * <br> & US - A - 4 218 584 <br> --- | 1,2, 7-9 | TECHNICAL FIELDS SEARCHED (Int. Cl.³) <br><br> A 61 B 7/04 <br> A 61 B 7/02 <br> A 61 B 7/00 |
| | US - A - 3 858 575 (E. ROSE) <br> * Abstract; column 1, lines 35-59; column 1, line 67 to column 2, line 66; figures 1 and 2 * <br> --- | 1 | |
| | GB - A - 1 159 127 (E. CHAGOURY) <br> * Page 1, lines 20-34; page 1, line 53 to page 2, line 53; figures 1-4 * <br> --- | 1,4,6-10 | |
| A | US - A - 3 160 708 (F.M. ANDRIES et al.) <br> * Column 1, lines 41-64; column 2, lines 47-72; figures 1,2 * <br> --- | 2,6-8 | CATEGORY OF CITED DOCUMENTS <br><br> X: particularly relevant <br> A: technological background <br> O: non-written disclosure <br> P: intermediate document <br> T: theory or principle underlying the invention <br> E: conflicting application <br> D: document cited in the application <br> L: citation for other reasons |
| | ./.. | | |

&: member of the same patent family, corresponding document

The present search report has been drawn up for all claims

| Place of search | Date of completion of the search | Examiner |
|---|---|---|
| The Hague | 21.05.1981 | BEAVEN |

EPO Form 1503.1  06.78

0034479

European Patent
Office

**EUROPEAN SEARCH REPORT**

Application number

EP 81 30 0594

- 2 -

| | **DOCUMENTS CONSIDERED TO BE RELEVANT** | | | CLASSIFICATION OF THE APPLICATION (Int. Cl.³) |
|---|---|---|---|---|
| Category | Citation of document with indication, where appropriate, of relevant passages | Relevant to claim | | |
| A | <u>US - A - 3 682 161</u> (V.F. ALIBERT)<br><br>* Abstract; column 5, line 51 to column 6, line 20; figures 1,2 * | 3,12 | | |
| | ---------- | | | |

TECHNICAL FIELDS
SEARCHED (Int. Cl.³)

EPO Form 1503.2   06.78